# EUROPEAN PATENT SPECIFICATION

(11) **EP 1 567 140 B1**
(45) Date of publication and mention of the grant of the patent: **02.07.2008**
(21) Application number: 03780056.2
(22) Date of filing: 26.11.2003
(51) Int. Cl.: A61K 31/167, A61K 31/44, A61P 11/00

(54) **SYNERGISTIC COMBINATION COMPRISING ROFLUMILAST AND (R,R)-FORMOTEROL**
SYNERGISTISCHE ZUSAMMENSETZUNG ENTHALTEND ROFLUMILAST UND (R,R)-FORMOTEROL
COMBINAISON SYNERGIQUE COMPRENANT DU ROFLUMILAST ET DU (R,R)-FORMOTEROL

(30) Priority: 27.11.2002 EP 02026504
(43) Date of publication of application: 31.08.2005
(73) Proprietor: Nycomed GmbH, 78467 Konstanz (DE)
(72) Inventor: BEUME, Rolf, 78465 Konstanz (DE); BUNDSCHUH, Daniela, 8272 Ermatingen (CH); MARX, Degenhard, 78345 Moos (DE); WEIMAR, Christian, 78467 Konstanz (DE); WOLLIN, Stefan-Lutz, 88709 Meersburg (DE)
(74) Representative: Wild, Robert
(86) International application number: PCT/EP2003/013266
(87) International publication number: WO 2004/047828

(56) References cited:
- WO-A-00/67741
- WO-A-01/13953
- WO-A-98/35683
- WO-A-02/066422
- WO-A-02/076933
- WO-A-02/088167
- US-A- 5 795 564
- US-B1- 6 288 118
- REID P: "ROFLUMILAST" CURRENT OPINION IN INVESTIGATIONAL DRUGS, CURRENT DRUGS, LONDON, GB, vol. 3, no. 8, August 2002 (2002-08), pages 1165-1170, XP001119630 ISSN: 0967-8298
- CAMPILLO N ET AL: "Novel bronchodilators in the treatment of asthma and COPD" EXPERT OPINION ON THERAPEUTIC PATENTS 2002 UNITED KINGDOM, vol. 12, no. 1, 2002, pages 53-63, XP002237401 ISSN: 1354-3776

## Description

### Field of application of the invention

The invention relates to the combination of certain known active compounds for therapeutic purposes. The substances used in the combination according to the invention are a known active compound from the PDE inhibitors class and an active compound from the β₂ adrenoceptor agonists class. Their combined use in the sense according to the invention for therapeutic purposes has not yet been described in the prior art.

### Prior art

International patent application WO01/13953 (US Patent 6,624,181) describes the combination of a compound from the class of PDE inhibitors with a compound from the class of β₂ adrenoceptor agonists for the treatment of respiratory tract disorders. - United States patent 6,288,118 generally describes the treatment of pulmonary diseases, such as chronic obstructive pulmonary disease or asthma, by administering a phosphodiesterase-4 inhibitor with a beta-adrenergic bronchodilator. - In Current Opinion in Investigational Drugs 2002 3(8): 1165-1170, the PDE4-inhibitor Roflumilast is described in detail. - In International patent application WO98/35683 a composition containing lung surfactant and Roflumilast is described. - In the review Expert Opin. Ther. Patents (2002) 12(1): 53-63 the patent literature during the period January 1998 to August 2001 concerning bronchodilators is analyzed, and exemplary compounds for the different substance classes are named, inter alia the PDE4-inhibitor Roflumilast. - United States patent 5,795,564 discloses a method and composition utilizing the pure (R, R) isomer of formoterol. - In International patent applications WO02/066422 and WO02/076933 certain new β₂ adrenoceptor agonists and their use in respiratory tract disorders are disclosed. - In International patent application WO00/67741 the pure (S, R) isomer of formoterol and its use is disclosed. - In International patent application WO02/088167 certain Androstane derivatives and combinations thereof with e.g. β₂ adrenoceptor agonists are described.

### Summary of the invention

The invention relates to compositions for preventing or reducing the onset of symptoms of pulmonary diseases, or treating or reducing the severity of pulmonary diseases. In particular it relates to compositions for treating pulmonary diseases mediated by phosphodiesterase 4 (PDE4) by administering a PDE4 inhibitor together with another pharmaceutically active agent, which affects pulmonary function. In this connection, it is the object of the present invention to make available a certain respiratory tract therapeutic which fulfills the following conditions:
- Pronounced antiinflammatory action
- Distinct bronchorelaxation and -dilatation
- Good oral availability, at least with respect to the PDE4 inhibitor
- Minor side effects
- Good suitability for long-term therapy
- Favorable influence on bronchial hyperreactivity.

It has now been found that the combined use of the PDE4 inhibitor roflumilast and of the β₂ adrenoceptor agonist R,R-formoterol outstandingly fulfills the abovementioned conditions, in particular in view of the fact that the combination of the two compounds acts synergistically, i. e. exhibits a greater than additive effect.

Accordingly, the invention relates in a first aspect to the manufacture of a medicament for preventing or reducing the onset of symptoms of a pulmonary disease, or treating or reducing the severity of a pulmonary disease by administering to a patient in need thereof an effective amount of roflumilast and R,R-formoterol either in a single combined form, separately, or separately and sequentially where the sequential administration is close in time, or remote in time.

The invention also relates to a composition for preventing or reducing the onset of symptoms of a pulmonary disease, or treating or reducing the severity of a pulmonary disease comprising an effective amount of roflumilast, an effective amount of R,R-formoterol and a pharmaceutically acceptable excipient.

### Detailed description of the invention

The combination therapy which is the subject matter of this invention comprises administering roflumilast with R,R-formoterol to prevent onset of a pulmonary disease event or to treat an existing condition. The two compounds may be administered together in a single dosage form. Or they may be administered in different dosage forms. They may be administered at the same time. Or they may be administered both close in time or remotely, such as where one drug is administered in the morning and the second drug is administered in the evening. The combination may be used prophylactically or after the onset of symptoms has occurred. In some instances the combination may be used to prevent the progression of a pulmonary disease or to arrest the decline of a function such as lung function.

The invention thus relates to the combined use of roflumilast and R,R-formoterol in preventing the symptoms of, or treating a respiratory tract disorder.

In the sense of the invention, the term "roflumilast" is understood to include the pharmacologically acceptable salts and the N-oxide of roflumilast, which can likewise be used according to the invention.

Correspondingly, the term "R,R-formoterol" is understood in connection with this invention to include the pharmacologically acceptable salts of R,R-formoterol.

It is understood that the active compounds mentioned can also be present, for example, in the form of their solvates, in particular in the form of their hydrates.

Suitable pharmacologically acceptable salts of roflumilast or R,R-formoterol are in particular watersoluble and water-insoluble acid addition salts with acids such as, for example, hydrochloric acid, hydrobromic acid, phosphoric acid, nitric acid, sulfuric acid, acetic acid, citric acid, D-gluconic acid, benzoic acid, 2-(4-hydroxybenzoyl)-benzoic acid, butyric acid, sulfosalicylic acid, maleic acid, lauric acid, malic acid, fumaric acid, succinic acid, oxalic acid, tartaric acid, embonic acid, stearic acid, toluenesulfonic acid, methanesulfonic acid or 1-hydroxy-2-naphthoic acid, the acids being employed in salt preparation - depending on whether it is a mono- or polybasic acid and depending on which salt is desired - in an equimolar quantitative ratio or one differing therefrom. A particularly preferred salt of R,R-formoterol is the fumarate.

Respiratory tract disorders which may be mentioned are in particular allergen- and inflammation-induced bronchial disorders (bronchitis, obstructive bronchitis, spastic bronchitis, allergic bronchitis, allergic asthma, bronchial asthma, COPD), which can be treated by the combination according to the invention also in the sense of a long-term therapy (if desired with appropriate adjustment of the dose of the individual components to the needs at the time, for example needs subject to seasonally related variations).

"Combined use" or "combination" within the meaning of the present invention is to be understood as meaning that the individual components can be administered simultaneously (in the form of a combination medicament), more or less simultaneously (from separate pack units) or in succession (directly in succession or else alternatively at a relatively large time interval) in a manner which is known per se and customary. As an example, one drug could be taken in the morning and one later in the day. Or in another scenario, one drug could be taken twice daily and the other once daily, either at the same time as one of the twice-a-day dosing occurred, or separately.

"Combined use" or "combination" within the meaning of the present invention is particularly to be understood as meaning that the two components act together in a synergistic manner.

R,R-formoterol is usually administered as an oral or nasal spray or aerosol, or as an inhaled powder. Usually R,R-formoterol is not administered systemically or by injection. Roflumilast can be administered orally or by inhalation (orally or internasally). This invention contemplates either co-administering both drugs in one delivery form such as an inhaler, which is putting both drugs in the same inhaler. Alternatively one can put roflumilast into pills and package them in a medicament pack with an inhaler that contains R,R-formoterol.

Within the meaning of the present invention, "use" can thus be understood as meaning primarily with respect to roflumilast the oral administration. In view of the synergistic effect of the combined use according to the invention, it is possible to use R,R-formoterol orally in a lower dose, avoiding thus the known side effects of orally administered R,R-formoterol in higher doses. With respect to R,R-formoterol, "use" is therefore, in accordance with the invention, understood primarily as meaning the oral administration, but it is also understood to mean topical application in inhalatory form. For inhalation, R,R-formoterol is preferably administered in the form of an aerosol, the aerosol particles of solid, liquid or mixed composition having a diameter of 0.5 to 10 µm, advantageously of 2 to 6 µm.

Aerosol generation can be carried out, for example, by pressure-driven jet atomizers or ultrasonic atomizers, but advantageously by propellant-driven metered aerosols or propellant-free administration of micronized active compounds from inhalation capsules.

The active compounds are dosed in an order of magnitude customary for the individual dose, it more likely being possible, on account of the individual actions, which are mutually positively influencing and reinforcing, to reduce the respective doses on the combined administration of the active compounds compared with the norm. For inhalation, R,R-formoterol is intended to be administered in a dose of preferably 10 to 50 µg per day by once, twice or three times daily administration.

Depending on the inhaler system used, in addition to the active compound the administration forms additionally contain the required excipients, such as, for example, propellants (e.g. Frigen in the case of metered aerosols), surface-active substances, emulsifiers, stabilizers, preservatives, flavorings, fillers (e.g. lactose in the case of powder inhalers) or, if appropriate, further active compounds.

For the purposes of inhalation, a large number of apparatuses are available with which aerosols of optimum particle size can be generated and administered, using an inhalation technique which is as right as possible for the patient. In addition to the use of adaptors (spacers, expanders) and pear-shaped containers (e.g. Nebulator®, Volumatic®), and automatic devices emitting a puffer spray (Autohaler®), for metered aerosols, in particular in the case of powder inhalers, a number of technical solutions are available (e.g. Diskhaler®, Rotadisk®, Turbohaler® or the inhaler described in European Patent Application EP 0 505 321), using which an optimal administration of active compound can be achieved.

In the case of the oral administration of R,R-formoterol, which is the preferred administration form in the combined use according to the invention, the daily dose is in the range from 20 to 120 µg per day by once, twice or three times daily oral administration.

In the case of the oral administration of roflumilast, which is the preferred administration form, the daily dose is in the range from 100 to 500 µg per day, preferably by once daily oral administration.

In case of medicaments which are intended for oral administration, the active ingredients roflumilast and/or R,R-formoterol are formulated to give medicaments according to processes known per se and familiar to the person skilled in the art. The active ingredients are employed as medicament, preferably in combination with suitable pharmaceutical excipients or vehicles, in the form of tablets, coated tablets, capsules, emulsions, suspensions or solutions, the active compound content advantageously being between 0.1 and 95% and, by the appropriate choice of the excipients and vehicles, it being possible to achieve a pharmaceutical administration form precisely tailored to the active compound(s) and/or to the desired onset of action (e.g. a sustained-release form or an enteric form). In case of a once daily oral administration of both roflumilast and R,R-formoterol in an oral single unit dosage form, R,R-formoterol is preferably formulated in such a way that it is released during a prolonged period of time.

The person skilled in the art is familiar on the basis of his/her expert knowledge with, which excipients or vehicles are suitable for the desired pharmaceutical formulations. In addition to solvents, gel-forming agents, tablet excipients and other active compound carriers, it is possible to use, for example, antioxidants, dispersants, emulsifiers, antifoams, flavor corrigents, preservatives, solubilizers, colorants or permeation promoters and complexing agents (e.g. cyclodextrins).

## Claims

1. A medicament comprising roflumilast, a pharmacologically acceptable salt of roflumilast or the N-oxide of roflumilast in combination with R,R-formoterol or a pharmacologically acceptable salt of R,R-formoterol.

2. Medicament according to claim 1, comprising roflumilast in combination with R,R-formoterol or a pharmacologically acceptable salt of R,R-formoterol.

3. Medicament according to claim 1, wherein the medicament is suited for oral administration.

4. Medicament according to claim 2, wherein the medicament is suited for oral administration.

5. Medicament according to claim 1, wherein the medicament is suited for administration by inhalation.

6. Medicament according to claim 2, wherein the medicament is suited for administration by inhalation.

7. Use of roflumilast, a pharmacologically acceptable salt of roflumilast or the N-oxide of roflumilast in combination with R,R-formoterol or a pharmacologically acceptable salt of R,R-formoterol in the manufacture of a medicament for preventing the symptoms of a respiratory tract disorder or for treating a respiratory tract disorder in humans.

8. Use according to claim 7, wherein roflumilast is used in combination with R,R-formoterol or a pharmacologically acceptable salt of R,R-formoterol in the manufacture of a medicament for preventing the symptoms of a respiratory tract disorder or for treating a respiratory tract disorder in humans.

9. Use according to claim 7, wherein roflumilast, a pharmacologically acceptable salt of roflumilast or the N-oxide of roflumilast is to be administered in a daily dosage of from 100 to 500 µg, and R,R-formoterol or a pharmacologically acceptable salt of R,R-formoterol is to be administered in a daily dosage of from 10 to 50 µg when administered by inhalation, and from 20 to 120 µg when administered orally.

10. Use according to claim 8, wherein roflumilast is to be administered in a daily dosage of from 100 to 500 µg, and R,R-formoterol or a pharmacologically acceptable salt of R,R-formoterol is to be administered in a daily dosage of from 10 to 50 µg when administered by inhalation, and from 20 to 120 µg when administered orally.

11. Use according to any one of claims 7 to 10, wherein the respiratory tract disorder is selected from bronchitis, obstructive bronchitis, spastic bronchitis, allergic bronchitis, allergic asthma, bronchial asthma or COPD.

12. Use according to any one of claims 7 to 10, wherein the respiratory tract disorder is bronchial asthma.

13. Use according to any one of claims 7 to 10, wherein the respiratory tract disorder is COPD.

## Patentansprüche

1. Medikament, enthaltend Roflumilast, ein pharmakologisch unbedenkliches Salz von Roflumilast oder das N-Oxid von Roflumilast in Kombination mit R,R-Formoterol oder einem pharmakologisch unbedenklichen Salz von R,R-Formoterol.

2. Medikament nach Anspruch 1, enthaltend Roflumilast in Kombination mit R,R-Formoterol oder einem pharmakologisch unbedenklichen Salz von R,R-Formoterol.

3. Medikament nach Anspruch 1, wobei das Medikament für die orale Verabreichung geeignet ist.

4. Medikament nach Anspruch 2, wobei das Medikament für die orale Verabreichung geeignet ist.

5. Medikament nach Anspruch 1, wobei das Medikament für die Verabreichung durch Inhalation geeignet ist.

6. Medikament nach Anspruch 2, wobei das Medikament für die Verabreichung durch Inhalation geeignet ist.

7. Verwendung von Roflumilast, einem pharmakologisch unbedenklichen Salz von Roflumilast oder dem N-Oxid von Roflumilast in Kombination mit R,R-Formoterol oder einem pharmakologisch unbedenklichen Salz von R,R-Formoterol bei der Herstellung eines Medikaments zur Prävention der Symptome einer Atemwegserkrankung oder zur Behandlung einer Atemwegserkrankung beim Menschen.

8. Verwendung nach Anspruch 7, wobei Roflumilast in Kombination mit R,R-Formoterol oder einem pharmakologisch unbedenklichen Salz von R,R-Formoterol bei der Herstellung eines Medikaments zur Prävention der Symptome einer Atemwegserkrankung oder zur Behandlung einer Atemwegserkrankung beim Menschen verwendet wird.

9. Verwendung nach Anspruch 7, wobei Roflumilast, ein pharmakologisch unbedenklichen Salz von Roflumilast oder das N-Oxid von Roflumilast in einer Tagesdosis von 100 bis 500 µg zu verabreichen ist und R,R-Formoterol oder ein pharmakologisch unbedenkliches Salz von R,R-Formoterol in einer Tagesdosis von 10 bis 50 µg zu verabreichen ist, wenn die Verabreichung durch Inhalation erfolgt, und von 20 bis 120 µg zu verabreichen ist, wenn die Verabreichung oral erfolgt.

10. Verwendung nach Anspruch 8, wobei Roflumilast in einer Tagesdosis von 100 bis 500 µg zu verabreichen ist und R,R-Formoterol oder ein pharmakologisch unbedenkliches Salz von R,R-Formoterol in einer Tagesdosis von 10 bis 50 µg zu verabreichen ist, wenn die Verabreichung durch Inhalation erfolgt, und von 20 bis 120 µg zu verabreichen ist, wenn die Verabreichung oral erfolgt.

11. Verwendung nach einem der Ansprüche 7 bis 10, wobei die Atemwegserkrankung ausgewählt ist aus Bronchitis, obstruktiver Bronchitis, spastischer Bronchitis, allergischer Bronchitis, allergischem Asthma, Bronchialasthma und COPD (Chronic Obstruktive Pulmonary Disease, chronisch-obstruktive Atemwegserkrankung).

12. Verwendung nach einem der Ansprüche 7 bis 10, wobei es sich bei der Atemwegserkrankung um Bronchialasthma handelt.

13. Verwendung nach einem der Ansprüche 7 bis 10, wobei es sich bei der Atemwegserkrankung um COPD handelt.

## Revendications

1. Médicament comprenant du roflumilast, un sel pharmacologiquement acceptable de roflumilast ou le N-oxyde de roflumilast en association avec du (R,R)-formotérol ou un sel pharmacologiquement acceptable de (R,R)-formotérol.

2. Médicament selon la revendication 1, comprenant du roflumilast en association avec du (R,R)-formotérol ou un sel pharmacologiquement acceptable de (R,R)-formotérol.

3. Médicament selon la revendication 1, le médicament étant approprié à une administration par voie orale.

4. Médicament selon la revendication 2, le médicament étant approprié à une administration par voie orale.

5. Médicament selon la revendication 1, le médicament étant approprié à une administration par inhalation.

6. Médicament selon la revendication 2, le médicament étant approprié à une administration par inhalation.

7. Utilisation de roflumilast, d'un sel pharmacologiquement acceptable de roflumilast ou du N-oxyde de roflumilast en association avec du (R,R)-formotérol ou un sel pharmacologiquement acceptable de (R,R)-formotérol, pour la fabrication d'un médicament destiné à la prévention des symptômes d'un trouble des voies respiratoires ou au traitement d'un trouble des voies respiratoires chez l'homme.

8. Utilisation selon la revendication 7, selon laquelle du roflumilast est utilisé en association avec du (R,R)-formotérol ou un sel pharmacologiquement acceptable de (R,R)-formotérol, pour la fabrication d'un médicament destiné à la prévention des symptômes d'un trouble des voies respiratoires ou au traitement d'un trouble des voies respiratoires chez l'homme.

9. Utilisation selon la revendication 7, selon laquelle le roflumilast, un sel pharmacologiquement acceptable de roflumilast ou le N-oxyde de roflumilast doit être administré en une dose journalière comprise entre 100 et 500 µg et le (R,R)-formotérol ou un sel pharmacologiquement acceptable de (R,R)-formotérol doit être administré en une dose journalière comprise entre 10 et 50 µg lorsque l'administration se fait par inhalation, et comprise entre 20 et 120 µg lorsque l'administration se fait par voie orale.

10. Utilisation selon la revendication 8, selon laquelle le roflumilast doit être administré en une dose journalière comprise entre 100 et 500 µg et le (R,R)-formotérol ou un sel pharmacologiquement acceptable de (R,R)-formotérol doit être administré en une dose journalière comprise entre 10 et 50 µg lorsque l'administration se fait par inhalation, et comprise entre 20 et 120 µg lorsque l'administration se fait par voie orale.

11. Utilisation selon l'une quelconque des revendications 7 à 10, selon laquelle le trouble des voies respiratoires est choisi parmi la bronchite, la bronchite obstructive, la bronchite spastique, la bronchite allergique, l'asthme allergique, l'asthme bronchique ou la BPCO.

12. Utilisation selon l'une quelconque des revendications 7 à 10, selon laquelle le trouble des voies respiratoires est l'asthme bronchique.

13. Utilisation selon l'une quelconque des revendications 7 à 10, selon laquelle le trouble des voies respiratoires est la BPCO.
